# EUROPÄISCHE PATENTSCHRIFT

(11) **EP 0 811 079 B1**
(45) Veröffentlichungstag und Bekanntmachung des Hinweises auf die Patenterteilung: **12.05.1999**
(21) Anmeldenummer: 96904706.7
(22) Anmeldetag: 23.02.1996
(51) Int. Cl.: C12Q 1/70

(54) **VERFAHREN ZUR FRÜHERKENNUNG VON HPV-ASSOZIIERTEN KARZINOMEN BZW. VON HOCHGRADIGEN, DURCH HPV-VERURSACHTE DYSPLASIEN**
EARLY RECOGNITION PROCESS OF HPV-ASSOCIATED CARCINOMAS OR HIGH-GRADE, HPV-CAUSED DYSPLASIAS
PROCEDE DE DETECTION PRECOCE DE CARCINOMES ASSOCIES AU VPH OU DE DYSPLASIES GRAVES PROVOQUEES PAR LE VPH

(30) Priorität: 24.02.1995 DE 19506561
(43) Veröffentlichungstag der Anmeldung: 10.12.1997
(73) Patentinhaber: Deutsches Krebsforschungszentrum Stiftung des öffentlichen Rechts, 69120 Heidelberg (DE)
(72) Erfinder: VON KNEBEL-DÖBERITZ, Magnus, D-69118 Heidelberg (DE); WÖRNER, Stefan, D-69123 Heidelberg (DE); EMMERICH, Florian, D-69120 Heidelberg (DE)
(74) Vertreter: Schüssler, Andrea, Dr.
(86) Internationale Anmeldenummer: DE9600306
(87) Internationale Veröffentlichungsnummer: WO9626293

(56) Entgegenhaltungen:
- EP-A- 0 373 352
- EP-A- 0 402 132
- EP-A- 0 466 367
- WO-A-88/06634
- WO-A-91/08312
- WO-A-94/26934
- JOURNAL OF GENERAL VIROLOGY, Bd. 71, Nr. PART 05, 1.Mai 1990, Seiten 1243-1246, XP000406161 CORNELISSEN M T E ET AL: "UNIFORMITY OF THE SPLICING PATTERN OF THE E6/E7 TRANSCRIPTS IN HUMAN PAPILLOMAVIRUS TYPE 16-TRANSFORMED HUMAN FIBROBLASTS, HUMAN CERVICAL PREMALIGNANT LESIONS AND CARCINOMAS"

## Beschreibung

Die Erfindung betrifft ein Verfahren zur Früherkennung von HPV-assoziierten Karzinomen bzw. von hochgradigen, durch HPV-verursachten Dysplasien.

Es ist bekannt, daß viele Menschen an persistierenden Infektionen durch humane Papillomviren (nachstehend mit HPVs bezeichnet) leiden. Ferner ist bekannt, daß mehr als 95 % aller Anogenitalkarzinome, insbesondere des Gebärmutterhalskrebs, und ein beachtlicher Prozentsatz der Karzinome im Mund/Rachenraum mit persisitierenden Infektionen durch sogenannte "Risikotypen" der HPVs assoziiert sind.

In der Krebsvorsorge bei der Frau werden daher z.B. Abstriche der Zervix uteri auf Veränderungen des Zellbildes untersucht, die durch persistierende Infektionen mit HPVs hervorgerufen werden. Solche Veränderungen werden in unterschiedliche Schweregrade eingeteilt. Lassen sich dysplastische Zellen nachweisen, wird in der Regel die Läsion durch Konisation der Zervix uteri entfernt. Seitdem dieses Verfahren regelmäßig zur Krebsfrüherkennung durchgeführt wird, hat die Inzidenz des invasiven Zervixkarzinoms in erheblichem Umfang abgenommen.

Dennoch weist vorstehendes Verfahren erhebliche Nachteile auf. Dies sind beispielweise: (1) Die Beurteilung des Zellbildes unterliegt subjektiven Einflüssen des Zytologen, die je nach seiner Erfahrung zu falsch positiven oder falsch negativen Ergebnissen führen können. (2) Durch die zytologische Beurteilung können Läsionen, die sich spontan zurückbilden können, nicht von Läsionen differenziert werden, die in ein invasives Karzinom übergehen werden. Bis zu 80 % der frühen Läsionen bilden sich spontan zurück. Aus Sicherheitsgründen werden diese Läsionen aber dennoch durch Konisation entfernt, um das Risiko einer möglichen Malignisierung zu vermeiden. Die Konisation selbst führt aber auch zu einer gewissen Morbidität, d.h. Zervixinsuffizienz, und damit verbundene geburtshilfliche Komplikationen. (3) Die zytologische Untersuchung ist in einigen Fällen nicht sensitiv genug, so daß trotz regelmäßiger zytologischer Kontrollen in einigen Fällen invasive Karzinome übersehen werden. EP373352 offenbart ein Verfahren zum Nachweis von Anwesenheit und Expression von humanen (Papillomaviren (HPV). Dies geschieht mit dem Einsatz von Amplimeren, die eine Spleißsequenz überdecken. Da diese Spleißsequenz von der Transkription integrierter HPV abstammt, weist dieses Verfahren die Anwesenheit und Expression von integrierten HPV nach. Der Zusammenhang zwischen in das Wirtsgenom integrierten HPV Sequenzen und der Transformation von infizierten Zellen zu Malignität ist auch anerkannt.

Jüngste Arbeiten weisen darauf hin, daß zur Entstehung von Karzinomen bzw. hochgradigen Dysplasien bei Zellen, die eine persistierende Infektion durch HPVs aufweisen, eine unkontrollierte Expression von HPV-Genen, insbesondere der Gene E6 und E7, notwendig ist. Auch wird darauf hingewiesen, daß in HPV-assoziierten Karzinomen HPV-Genome oder zumindest die Gene E6 und E7 in die Zell-DNA integriert sind und zusammen mit zellulären Sequenzen exprimiert werden.

Dies könnte bedeuten, daß bereits für die unkontrollierte Expression vorstehender Gene diese zusammen mit zellulären Sequenzen exprimiert werden müssen. Ein solches Expressionsprodukt könnte dann geeignet sein, die Entstehung von HPV-assoziierten Karzinomen bzw. von hochgradigen, durch HPV-verursachten Dysplasien frühzeitig aufzuzeigen.

Die Anmelderin hat vorstehendes untersucht und gefunden, daß das angesprochene Expressionsprodukt für die unkontrollierte Expression der genannten Gene steht und sich so zum frühzeitigen Nachweis von HPV-assoziierten Karzinomen bzw. von hochgradigen, durch HPV-verursachten Dysplasien eignet. Als günstig hat sich gezeigt, das Expressionsprodukt in Form einer mRNA nachzuweisen, die HPV- und zelluläre Sequenzen aufweist. Ein hierzu geeignetes Verfahren umfaßt folgende Verfahrensschritte:
(a) Entnahme und Aufbereitung einer Körperprobe,
(b) Isolierung von mRNA aus der Körperprobe von (a),
(c) Übersetzung der mRNA von (b) in cDNA mit einem für eine reverse Transkription üblichen Primer,
(d) Amplifikation der cDNA von (c) durch eine PCR-Reaktion mit einem HPV-Primer (5'-Primer) und einem, Sequenzen des Primers von (c) aufweisenden Primer (3'-Primer),
(e) Spaltung der amplifizierten cDNA von (d) mit einer an der 5'-Seite der HPV-Polyadenylierungssequenz spaltenden Endonuklease,
(f) Amplifikation der nicht-gespaltenen cDNA von (e) mit den Primern von (d) oder mit "nested" Primern, und
(g) Nachweis der amplifizierten cDNA von (f).

Eine Körperprobe, in der eine, HPV- und zelluläre Sequenzen aufweisende mRNA nachgewiesen werden soll, wird dem Patienten entnommen. Geeignet sind hierzu u.a. ein Abstrich, ein Organpunktat bzw. eine Biopsie, Blut, Sputum, Urin, Stuhl, Liquor, Galle, Lymphflüssigkeit und ein gastrointestinales Sekret, wobei ein Abstrich bevorzugt ist.

Die Entnahme und Aufarbeitung der Körperprobe, vorzugsweise eines Abstrichs, erfolgt in üblicher Weise. Ebenso wird die mRNA aus der Körperprobe nach üblichen Verfahren isoliert. Günstig ist es, einen käuflichen Extraktionskit, z.B. GlassMax, Gibco BRL, zu verwenden. Kontaminierende DNA in der mRNA-Präparation wird durch übliche DNase-Verdauung entfernt.

Die erhaltene mRNA wird einer reversen Transkription unterzogen, wobei ein üblicher Primer verwendet wird. Vorzugsweise weist der Primer eine der folgenden Sequenzen auf:

5′- GAC TCG AGT CGA CAT CGA TTT TTT TTT TTT TTT TT - 3′;

5'- GAC TCG AGT CGA CAT CGA GAC GTC GGA GTG CGA GCA TCG ATT TTT
TTT TTT TTT TTT - 3'

Für die reverse Transkription kann eine übliche reverse Transkriptase, vorzugsweise eine MMLV-reverse Transkriptase (z.B. Superskript II, Gibco BRL) verwendet werden. Ein bevorzugter Reaktionsansatz umfaßt folgendes:
4 µℓ 5 x Reaktionspuffer
2 µℓ 0,1 M DTT
1 µℓ 10 mM dNTPs
1 µℓ 25 pmol/µℓ Primer
200 Units reverse Transkriptase
circa 1 µg RNA
Ansatz ad 20 µℓ, Reaktionsbedingungen:
10 min, 70°C; 60 min, 42°C; 30 min, 52°C.

Die erhaltene cDNA wird einer Polymerase-Kettenreaktion (PCR-Reaktion) unterzogen, wobei es günstig ist, eine käufliche Taq-Polymerase (z.B. Gibco BRL) zu verwenden. Als Primer werden ein HPV-Primer (5'-Primer), vorzugsweise aus der E6-E7-Region, und ein Primer (3'-Primer) verwendet, der Sequenzen, vorzugsweise aus dem 5'-Bereich, eines vorstehenden für die reverse Transkription verwendeten Primers aufweist.

Als 5'-Primer für die E6-E7 Region von häufig vorkommenden Risikotypen von HPV eignen sich besonders jene folgender Sequenzen:

HPV 16: 5′- CGG ACA GAG CCC ATT ACA AT - 3′ nt 701-nt 720
HPV 18: 5′- TAG AAA GCT CAG CAG ACG ACC - 3′ nt 816-nt 836
HPV 31: 5′- TAT GAG CAA TTA CCC GAC AGC - 3′ nt 632-nt 642
HPV 33: 5′- TTT ATA TCC TGA ACC AAC TGA nt 614-nt 642
CCT ATA CT - 3′
HPV 35: 5′- TAG ATT TGG AAC CCG AGG C - 3′ nt 599-nt 618
HPV 39: 5′- TCA CGA GCA ATT AGG AGA GTC A - 3′ nt 675-nt 696
HPV 52: 5′- GCA ACC TGA AAC AAC TGA CCT AC - 3′ nt 597-nt 608

Als 3'-Primer eignet sich besonders jener folgender Sequenz:

5′- GAC TCG AGT CGA CAT CG - 3′.

Erfindungsgemäß können ein oder mehrere 5'-Primer und/oder 3'-Primer in die PCR-Reaktion eingesetzt werden. Liegen mehrere 5'-Primer vor, können diese für einen HPV-Typ oder für mehrere HPV-Typen sein.

Die PCR-Reaktion wird unter Standardbedingungen durchgeführt. Dies sind z.B. die folgenden Bedingungen:
10 µℓ 10 x Taq-Polymerasepuffer
10 µℓ 2 mM dNTPs
3 µℓ 50 mM MgCℓ₂
1 µℓ 25 pmol/µℓ 5'-Primer
1 µℓ 25 pmol/µℓ 3'-Primer
1 Unit Taq-DNA-Polymerase
2 µℓ cDNA (aus reverser Transkription)
ad 100 µℓ
Reaktionstemperaturen: 1,5 min, 93°C; 30 sec, 93°C; 30 sec, x°C (x = unterschiedliche Annealingtemperatur je nach Primer), 2 min, 72°C, wiederholt in 30 Zyklen; finale Elongation 6 min, 72°C. Die Annealingtemperaturen werden entsprechend der Thermodynamik der verwendeten Primer gewählt. Dies ist für den Fachmann ein üblicher Schritt.

Zur Kontrolle vorstehender Anreicherung von mRNA und deren Übersetzung in cDNA empfiehlt es sich, eine GAPDH-PCR-Reaktion durchzuführen. Diese erfolgt unter den vorstehenden Bedingungen. Als Primer werden z.B. verwendet:

GAPDH (forward):  5'- CAT CTC TGC CCC CTC TGC TGA - 3'
GAPDH (reverse):  5'- GGA TGA CCT TGC CCA CAG CCT - 3'

Der Nachweis amplifizierter GAPDH-Sequenzen erfolgt in üblicher Weise, z.B. durch Southern Blot-Hybridisierung mit einem markierten, für GAPDH spezifischen Oligonukleotid. Dieses Oligonukleotid kann z.B. folgende Sequenz aufweisen:

5'- CTC TCC AGA ACA TCA TCC CTG - 3'

Durch vorstehende Reaktionen, die nicht die Kontrolle betreffen, werden cDNAs erhalten, die auf mRNAs zurückgehen, welche HPV-E6-E7- und Polyadenylierungssequenzen aufweisen. Solche mRNAs können von episomalen HPVs stammen, die in Zellen mit einer persistierenden HPV-Infektion vorliegen. Andererseits können die mRNAs auch von in der Zell-DNA integrierten HPVs stammen und zelluläre Sequenzen aufweisen. Zum Nachweis letzterer mRNAs werden die vorstehenden cDNAs mit einem Restriktionsenzym gespalten, das an der 5'-Seite der HPV-Polyadenylierungssequenz spaltet. Damit werden nur jene cDNAs gespalten, die von episomalen HPVs erhalten sind. Die anderen cDNAs, die von integrierten HPVs erhalten sind, werden nicht gespalten, da ihnen die Schnittstelle für das Restriktionsenzym fehlt. Dies ist auf die Rekombination zwischen viralen und zellulären Sequenzen zurückzuführen, durch die Sequenzen an der 5'-Seite der HPV-Polyadenylierungssequenz nicht co-transkribiert werden (vgl. Fig.).

Als Restriktionsenzyme für vorstehende Risikotypen von HPV eiqnen sich insbesondere die folgenden:

| | |
|---|---|
| HPV 16 | Alw NI |
| HPV 18 | Nde I |
| HPV 31 | BsmBI |
| HPV 33 | Sca I |
| HPV 39 | Hph I |
| HPV 52 | BstE II |

Die nicht-gespaltenen cDNAs werden einer erneuten PCR-Reaktion unterzogen. Hierzu können die Primer verwendet werden, die vorstehend für die erste PCR-Reaktion eingesetzt wurden.

Günstig ist es auch, nested Primer" zu verwenden, welche die zu amplifizierenden cDNAs einengen. Solche Primer können sowohl als 5'-Primer wie auch als 3'-Primer eingesetzt werden. Auch können Kombinationen aus Primern, die für die erste PCR-Reaktion eingesetzt wurden, und "nested" Primern günstig sein.

Als "nested" 5'-Primer für die E6-E7 Region vorstehender HPV-Risikotypen eignen sich besonders jene folgender Sequenzen:

HPV 16: 5′- CCT TTT GTT GCA AGT GTG ACT nt 728-nt 753
CTA CG - 3′
HPV 18: 5′- GAG CAT TCC AGC AGC TGT TTC nt 840-nt 864
TGA A - 3′
HPV 31: 5′- CAC GAG CAC ACA AGT AGA TAT nt 766-nt 798
TCG CAT ATT GCA - 3′
HPV 33: 5′- TCA ACA GTA CAG CAA GTG ACC nt 778-nt 807
TAC GAA CCA - 3′
HPV 35: 5′- AGA GGA GGA GGA AGA TAC TAT nt 657-nt 687
TGA CGG TCC A - 3′
HPV 39: 5′- GTT AAT CAC CAA CAT CAA CTA nt 727-nt 759
CTA GCC AGA CGG - 3′
HPV 52: 5′- CGG CCA GAT GGA CAA GCA GAA nt 676-nt 698
CA - 3′

Als nested 3'Primer eignet sich besonders jener folgender Sequenz:

5'- GAC GTC GGA GTG CGA GCA TCG - 3'.

Die PCR-Reaktion (zweite PCR-Reaktion) wird ebenfalls unter Standardbedingungen durchgeführt. Dies sind z.B. folgende Bedingungen:
10 µℓ 10 x Taq-Polymerasepuffer
10 µℓ 2 mM dNTPs
3 µℓ 50 mM MgCℓ₂
1 µℓ 25 pmol/µℓ 5'-Primer
1 µℓ 25 pmol/µℓ 3'-Primer
1 Unit Taq-DNA-Polymerase
2 µℓ cDNA (aus reverser Transkription)
ad 100 µℓ
Reaktionstemperaturen: 1,5 min, 93°C; 30 sec, 93°C; 30 sec, x°C (x = unterschiedliche Annealingtemperatur je nach Primer), 2 min, 72°C, wiederholt in 30 Zyklen; finale Elongation 6 min, 72°C. Die Annealingtemperaturen werden entsprechend der Thermodynamik der verwendeten Primer gewählt. Dies ist für den Fachmann ein üblicher Schritt.

Der Nachweis der amplifizierten cDNAs erfolgt in üblicher Weise, z.B. durch Southern Blot-Hybridisierung mit markierten, für die einzelnen HPV-Typen spezifischen Oligonukleotiden. Dies können z.B. die verwendeten Primer bzw. nested Primer" sein.

Das erfindungsgemäße Verfahren zeichnet sich durch hohe Sensitivität und Selektivität aus. Es ermöglicht, geringste Mengen an HPV-infizierten Zellen aufzuspüren, die aufgrund ihrer genetischen Veränderung zu HPV-assoziierten Karzinomen auswachsen würden. Das erfindungsgemäße Verfahren eignet sich daher bestens, zur Früherkennung von HPV-assoziierten Karzinomen, bzw. von hochgradigen, durch HPV-verursachten Dysplasien eingesetzt zu werden.

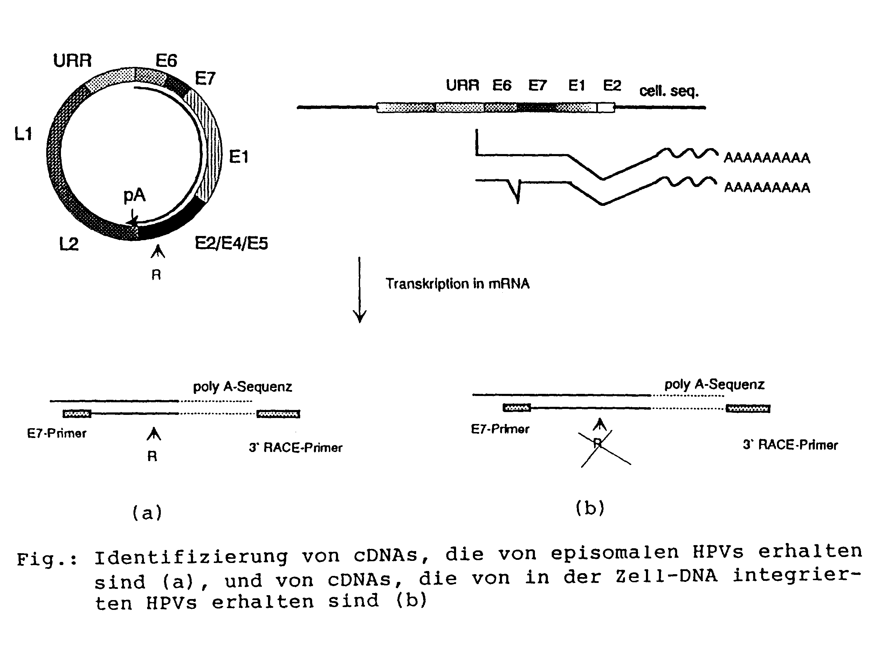

### Kurze Beschreibung der Zeichnung

Die Figur zeigt die Identifizierung von cDNAs, die von episomalen HPVs erhalten sind (a), und von cDNAs, die von in der Zell-DNA integrierten HPVs erhalten sind (b).

Die vorliegende Erfindung wird durch das folgende Beispiel erläutert.

### Beispiel: Nachweis einer, HPV 18- und zelluläre Sequenzen aufweisenden mRNA in einem Abstrich

Aus einem Zervix uteri-Abstrich von einer Patientin wurde mittels des käuflichen Extraktionskits GlassMax mRNA isoliert und angereichert. Die mRNA wurde einer reversen Transkription unterzogen, wobei als Primer ein solcher mit der Sequenz 5'- GAC TCG AGT CGA CAT CGA TTT TTT TTT TTT TTT TT - 3' verwendet wurde.

Der Reaktionsansatz war wie folgt:
4 µℓ 5 x Reaktionspuffer
2 µℓ 0,1 M DTT
1 µℓ 10 mM dNTPs
1 µℓ 25 pmol/µℓ Primer
200 Units reverse Transkriptase des MMLV
circa 1 µg vorstehende mRNA
Ansatz ad 20 µℓ
Reaktionsbedingungen: 10 min, 70°C; 60 min, 42°C; 30 min, 52°C.

Die erhaltene cDNA wurde einer PCR-Reaktion unterzogen. Als 5'-Primer wurde einer für HPV 18 geeignete Primer mit der Sequenz 5'- TAG AAA GCT CAG CAG ACG ACC - 3'verwendet. Als 3'-Primer wurde einer mit der Sequenz 5'- GAC TCG AGT CGA CAT CG - 3' verwendet. Der PCR-Reaktionsansatz war wie folgt:
10 µℓ 10 x Taq-Polymerasepuffer
10 µℓ 2 mM dNTPs
3 µℓ 50 mM MgCℓ₂
1 µℓ 25 pmol/µℓ 5'- Primer (HPV-18 Primer)
1 µℓ 25 pmol/µℓ 3'-Primer
1 Unit Taq-DNA-Polymerase
2 µℓ cDNA (aus reverser Transkription)
Ansatz ad 100 µℓ
Reaktionstemperaturen: 1,5 min, 93°C; 30 sec, 93°C; 30 sec, 56°C (Annealingtemperatur), 2 min, 72°C, wiederholt in 30 Zyklen; finale Elongation 6 min, 72°C.

Zur Kontrolle wurde vorstehende cDNA einer GAPDH-PCR-Reaktion unterzogen. Diese wurde unter den vorstehenden Bedingungen durchgeführt, wobei als Primer verwendet wurden:

GAPDH (forward): 5′- CAT CTC TGC CCC CTC TGC TGA - 3′
GAPDH (reverse): 5′- GGA TGA CCT TGC CCA CAG CCT - 3′

Die amplifizierte cDNA (Kontrolle) wurde einer Southern Blot-Hybridisierung unterzogen, wobei ein 32p-5'-markiertes Oligonukleotid mit der Sequenz 5'- CTC TCC AGA ACA TCA TCC CTG - 3' als Probe verwendet wurde. Die Hybridisierungstemperatur lag etwa 5°C unter der Schmelztemperatur des Oligonukleotids.

Es wurde eine amplifizierte DNA nachgewiesen.

Die mit dem HPV 18-Primer amplifizierte DNA wurde einer Restriktionsspaltung mit Nde I unterzogen. Der Reaktionsansatz war wie folgt:
1 µg DNA
2 µl 10x NdeI-Puffer
2 µl NdeI (10 U)
Ansatz ad 20 µl
Reaktionsbedingungen: 2 Std. 37°C

Die gespaltene DNA wurde einer erneuten PCR-Reaktion unter folgenden Bedingungen unterzogen:
10 µℓ 10 x Taq-Polymerasepuffer
10 µℓ 2 mM dNTPs
3 µℓ 50 mM MgCℓ₂
1 µℓ 25 pmol/µℓ "nested" HPV 18-Primer: 5'- GAG CAT TCC AGC AGC TGT TTC TGA A - 3'
1 µℓ 25 pmol/µℓ "nested" 3'-Primer: 5'- GAC GTC GGA GTG CGA GCA TCG - 3'
1 Unit Taq-DNA-Polymerase
2 µℓ cDNA (aus reverser Transkription)
ad 100 µℓ
Reaktionstemperaturen: 1,5 min, 93°C; 30 sec, 93°C; 30 sec, 56°C (Annealingtemperatur), 2 min, 72°C, wiederholt in 30 Zyklen; finale Elongation 6 min, 72°C.

Die amplifizierte cDNA wurde einer Southern Blot-Hybridisierung unterzogen, wobei ein 32p-5'-markiertes Oligonukleotid mit der Sequenz 5'- CAA TAC TGT CTT GCA ATA TAC - 3' als Probe verwendet wurde. Die Hybridisierungstemperatur lag etwa 5°C unter dem Schmelzpunkt des Oligonukleotids.

Es wurde eine amplifizierte cDNA nachgewiesen, die sich von einer, HPV- und zelluläre Sequenzen aufweisenden mRNA ableitete.

## Patentansprüche

1. Verfahren zum Nachweis einer, HPV- und zelluläre Sequenzen aufweisenden mRNA in einer Körperprobe, umfassend die folgenden Verfahrensschritte:
(a) Entnahme und Aufbereitung einer Körperprobe,
(b) Isolierung von mRNA aus der Körperprobe von (a),
(c) Übersetzung der mRNA von (b) in cDNA mit einem für eine reverse Transkription üblichen Primer,
(d) Amplifikation der cDNA von (c) durch eine PCR-Reaktion mit einem HPV-Primer (5'-Primer) und einem, Sequenzen des Primers von (c) aufweisenden Primer (3'-Primer),
(e) Spaltung der amplifizierten cDNA von (d) mit einer, an der 5'-Seite der HPV-Polyadenylierungssequenz spaltenden Endonuklease,
(f) Amplifikation der nicht-gespaltenen cDNA von (e) mit den Primern von (d) oder mit "nested" Primern, und
(g) Nachweis der amplifizierten cDNA von (f).

2. Verfahren nach Anspruch 1, dadurch gekennzeichnet, daß die Körperprobe ein Abstrich, ein Organpunktat bzw. eine Biopsie, Blut, Sputum, Urin, Stuhl, Liquor, Galle, Lymphflüssigkeit und/oder ein gastrointestinales Sekret ist.

3. Verfahren nach Anspruch 1 oder 2, dadurch gekennzeichnet, daß der 5'-Primer ein Primer für HPV-Risikotypen, wie HPV 16, 18, 31, 33, 35, 39 oder 52 ist.

4. Verfahren nach einem der Ansprüche 1-3, dadurch gekennzeichnet, daß mehrere 5'-Primer und/oder 3'-Primer verwendet werden.

5. Verfahren nach einem der Ansprüche 1-4, dadurch gekennzeichnet, daß in Schritt (d) ein oder mehr verwendet werden
als 5'-Primer: 
HPV 16: 5′- CGG ACA GAG CCC ATT ACA AT - 3′
HPV 18: 5′- TAG AAA GCT CAG CAG ACG ACC - 3′
HPV 31: 5′- TAT GAG CAA TTA CCC GAC AGC - 3′
HPV 33: 5′- TTT ATA TCC TGA ACC AAC TGA CCT ATA CT - 3′
HPV 35: 5′- TAG ATT TGG AAC CCG AGG C - 3′
HPV 39: 5′- TCA CGA GCA ATT AGG AGA GTC A - 3′
HPV 52: 5′- GCA ACC TGA AAC AAC TGA CCT AC - 3′
 als 3'-Primer:

5'- GAC TCG AGT CGA CAT CG - 3'

6. Verfahren nach einem der Ansprüche 1-4, dadurch gekennzeichnet, daß in Schritt (f) ein oder mehr verwendet werden
als "nested" 5'-Primer: 
HPV 16: 5′- CCT TTT GTT GCA AGT GTG ACT CTA CG - 3′
HPV 18: 5′- GAG CAT TCC AGC AGC TGT TTC TGA A - 3′
HPV 31: 5′- CAC GAG CAC ACA AGT AGA TAT TCG CAT
ATT GCA - 3′
HPV 33: 5′- TCA ACA GTA CAG CAA GTG ACC TAC GAA CCA - 3′
HPV 35: 5′- AGA GGA GGA GGA AGA TAC TAT TGA CGG
TCC A - 3′
HPV 39: 5′- GTT AAT CAC CAA CAT CAA CTA CTA GCC
AGA CGG -′3
HPV 52: 5′- CGG CCA GAT GGA CAA GCA GAA CA - 3′
 als "nested" 3'-Primer:

5′- GAC GTC GGA GTG CGA GCA TCG - 3′

7. Verwendung des Verfahrens nach einem der Ansprüche 1-6 zur Früherkennung HPV-assoziierter Karzinome bzw. hochgradiger, durch HPV-verursachter Dysplasien.

## Claims

1. A method of detection of an mRNA having HPV and cellular sequences in a body sample, comprising the following steps:
(a) taking and preparation of a body sample,
(b) isolation of mRNA from the body sample of (a),
(c) translation of the mRNA of (b) into CDNA using a primer common for a reverse transcription,
(d) amplification of the cDNA of (c) by a PCR reaction with an HDV primer (5' primer) and a primer (3) primer) having sequences of the primer of (c),
(e) cleavage of the amplified cDNA of (d) with an endonuclease cleaving on the 5' side of the HPV polyadenylation sequence,
(f) amplification of the non-cleaved cDNA of (e) with the primers of (d) or with "nested" primers, and
(g) detection of the amplified cDNA of (f).

2. The method according to claim 1, characterized in that the body sample is a smear or surface biopsy, an organ punctate and a biopsy, respectively, blood, sputum, urine, stool, liquor, bile, lymph and/or a gastrointestinal secretion.

3. The method according to claim 1 or 2, characterized in that the 5' primer is a primer for high-risk HPV types, such as HPV 16, 18, 31, 33, 35, 39 or 52.

4. The method according to any one of claims 1 to 3, characterized in that several 5' primers and/or 3' primers are used.

5. The method according to any one of claims 1 to 4, characterized in that in step (d) one or more of the following are used
as 5' primers: 
HPV 16: 5′- CGG ACA GAG CCC ATT ACA AT - 3′
HPV 18: 5′- TAG AAA GCT CAG CAG ACG ACC - 3′
HPV 31: 5′- TAT GAG CAA TTA CCC GAC AGC - 3′
HPV 33: 5′- TTT ATA TCC TGA ACC AAC TGA CCT ATA CT - 3′
HPV 35: 5′- TAG ATT TGG AAC CCG AGG C - 3′
HPV 39: 5′- TCA CGA GCA ATT AGG AGA GTC A - 3′
HPV 52: 5′- GCA ACC TGA AAC AAC TGA CCT AC - 3′
 as 3' primer:

5'- GAC TCG AGT CGA CAT CG - 3'

6. The method according to any one of claims 1 to 4, characterized in that in step (f) one or more of the following are used
as "nested" 5' primers: 
HPV 16: 5′- CCT TTT GTT GCA AGT GTG ACT CTA CG - 3′
HPV 18: 5′- GAG CAT TCC AGC AGC TGT TTC TGA A - 3′
HPV 31: 5′- CAC GAG CAC ACA AGT AGA TAT TCG CAT
ATT GCA - 3′
HPV 33: 5′- TCA ACA GTA CAG CAA GTG ACC TAC GAA CCA - 3′
HPV 35: 5′- AGA GGA GGA GGA AGA TAC TAT TGA CGG
TCC A - 3′
HPV 39: 5′- GTT AAT CAC CAA CAT CAA CTA CTA GCC
AGA CGG -′3
HPV 52: 5′- CGG CCA GAT GGA CAA GCA GAA CA - 3′
 as "nested" 3' primer:

5'- GAC GTC GGA GTG CGA GCA TCG - 3'

7. Use of the method according to any one of claims 1 to 6 for the early detection of HPV-associated carcinomas and extreme dysplasias caused by HPV, respectively.

## Revendications

1. Procédé pour détecter un ARN messager qui possède des séquences cellulaires et des séquences de HPV dans un échantillon organique, comportant les étapes consistant à :
(a) prélever et préparer un échantillon organique,
(b) isoler l'ARN messager de l'échantillon organique obtenu en (a),
(c) traduire l'ARN messager obtenu en (b) en ADN complémentaire avec une amorce classique de transcription reverse,
(d) amplifier l'ADN complémentaire obtenu en (c) par une réaction de polymérisation en chaîne (PCR) avec une amorce HPV (amorce 5') et une amorce (amorce 3') comportant des séquences de l'amorce de l'étape (c),
(e) morceler l'ADN complémentaire amplifié obtenu en (d) avec une endonucléase morcelant du côté 5' de la séquence de signal de polyadénylation de HPV,
(f) amplifier l'ADN complémentaire non morcelé obtenu en (e) avec les amorces de l'étape (d) ou avec des amorces "emboîtées", et
(g) détecter l'ADN complémentaire amplifié obtenu en (f).

2. Procédé selon la revendication 1, caractérisé en ce que l'échantillon organique est un prélèvement, une ponction d'organe ou une biopsie, du sang, du crachat, de l'urine, des fèces, du liquide céphalo-rachidien, de la bile, de la lymphe et/ou une sécrétion gastro-intestinale.

3. Procédé selon la revendication 1 ou 2, caractérisé en ce que l'amorce 5' est une amorce pour les types à risque du HPV, comme HPV 16, 18, 31, 33, 35, 39 ou 52.

4. Procédé selon l'une quelconque des revendications 1 à 3, caractérisé en ce qu'on utilise plusieurs amorces 5' et/ou amorces 3'.

5. Procédé selon l'une quelconque des revendications 1 à 4, caractérisé en ce qu'on utilise à l'étape (d) une ou plusieurs séquences parmi 
HPV 16 : 5'-CGG ACA GAG CCC ATT ACA AT-3'
HPV 18 : 5'-TAG AAA GCT CAG CAG ACG ACC-3'
HPV 31 : 5'-TAT GAG CAA TTA CCC GAC AGC-3'
HPV 33 : 5'-TTT ATA TCC TGA ACC AAC TGA CCT ATA CT-3'
HPV 35 : 5'-TAG ATT TGG AAC CCG AGG C-3'
HPV 39 : 5'-TCA CGA GCA ATT AGG AGA GTC A-3'
HPV 52 : 5'-GCA ACC TGA AAC AAC TGA CCT AC-3',
 comme amorce 5', 5'-GAC TCG AGT CGA CAT CG-3', comme amorce 3'.

6. Procédé selon l'une quelconque des revendications précédentes, caractérisé en ce qu'on utilise à l'étape (f) une ou plusieurs séquences parmi 
HPV 16 : 5'-CCT TTT GTT GCA AGT GTG ACT CTA CG -3'
HPV 18 : 5'-GAG CAT TCC AGC AGC TGT TTC TGA A-3'
HPV 31 : 5'-CAC GAG CAC ACA AGT AGA TAT TCG CAT ATT
GCA-3'
HPV 33 : 5'-TCA ACA GTA CAG CAA GTG ACC TAC GAA CCA-3'
HPV 35 : 5'-AGA GGA GGA GGA AGA TAC TAT TGA CGG TCC
A -3'
HPV 39 : 5'-GTT AAT CAC CAA CAT CAA CTA CTA GCC AGA
CGG-3'
HPV 52 : 5'-CGG CCA GAT GGA CAA GCA GAA CA-3',
 comme amorce emboîtée" 5', 5'-GAC GTC GGA GTG CGA GCA TCG-3', comme amorce "emboîtée" 3'.

7. Utilisation du procédé selon l'une quelconque des revendications 1 à 6 pour la détection rapide de carcinomes associés au HPV ou de dysplasies de haut degré causées par HPV.
